Europäisches Patentamt

European Patent Office

Office européen des brevets

(19)

(11) Publication number: 0 243 338 A2

# (12) EUROPEAN PATENT APPLICATION

(21) Application number: 87870055.8

(22) Date of filing: 23.04.87

(51) Int. Cl.⁴: **C 12 N 15/00**
C 12 N 1/16, C 12 N 1/20,
C 12 N 9/20

(30) Priority: 25.04.86 GB 8610230

(43) Date of publication of application:
28.10.87 Bulletin 87/44

(84) Designated Contracting States:
AT BE CH DE ES FR IT LI LU NL SE

(71) Applicant: LABOFINA S.A.
52, rue de l'Industrie
B-1040 Bruxelles (BE)

(72) Inventor: Vandamme, Etienne
62, avenue de la Tenderie
B-1170 Bruxelles (BE)

Schanck-Brodrück, Karin H.
60, rue de la Neuville
B-1348 Louvain-La-Neuve (BE)

Colson, Charles
26a, rue des Frères Poels
B-1320 Dion Valmont (BE)

Hanotier, Jacques D.V.
5B rue de Caturia
B-1338 Lasne (BE)

(54) DNA segment coding for a specific lipase, vectors for the expression thereof, microorganisms transformed by these vectors and use of these microorgenisms for the production of the lipase.

(57) DNA segment coding for a lipase which catalyses the hydrolysis of triglycerides with a specificity for fatty acids containing cis-9 unsaturation, characterized in that said segment comprises a sequence selected from the group comprising the following nucleotide sequence : and subfragments and mutants thereof coding for lipases having retained substantially the same specificity.

```
         10        20        30        40        50        60        70         80
ATGAAATTTGTAAAAAGAAGGATCATTGCACTTGTAACAATTTTGATGCTGTCTGTTACATCGCTGTTTGCGTTGCAGCC

         90       100       110       120       130       140       150       160
GTCAGCAAAAGCCGCTGAACACAATCCAGTCGTTATGGTTCACGGTATTGGAGGGGCACCATTCAATTTTGCGGGAACTA

        170       180       190       200       210       220       230       240
AGAGCTATCCCGTATCTCAGGGCTGGTCGCGGGACAAGCTGTATGCAGTTGATTTTTGGGACAAGACAGGCACAAATTAT

        250       260       270       280       290       300       310       320
AACAATGGACCGGTATTATCGCGATTTGTGCAAAAGGTTTTAGATGAAACGGGTGCGAAAAAAGTGGATATTGTCGCTCG

        330       340       350       360       370       380       390       400
CAGCATGGGGGGCGCGAACACACTTTACTACATAAAAAATCTGGACGGCGGAAATAAAGTTGCAAACGTCGTGACGCTTG

        410       420       430       440       450       460       470       480
GCGGCGCGAACCGTTTGACGACAGGCAAGGCGCTTCCGGGAACAGATCCAAATCAAAAGATTTTATACACATCCATTTAC

        490       500       510       520       530       540       550       560
AGCAGTGCCGATATGATTGTCATGAATTACTTATCAAGATTAGATGGTGCTAGAAACGTTCAAATCCATGGCGTTGGACA

        570       580       590       600       610       620       630       640
CATCGGCCTTCTGTACAGCAGCCAAGTCAACAGCCTGATTAAAGAAGGGCTGAACGGCGGGGGGCCAGAATACGAATTAA
```

EP 0 243 338 A2

**Description**

DNA SEGMENT CODING FOR A SPECIFIC LIPASE, VECTORS FOR THE EXPRESSION THEREOF, MICROORGANISMS TRANSFORMED BY THESE VECTORS AND USE OF THESE MICROORGANISMS FOR THE PRODUCTION OF THE LIPASE

TECHNICAL FIELD

The present invention relates to a DNA segment coding for a lipase which catalyses the hydrolysis of triglycerides with a specificity for fatty acids containing cis-9 unsaturation. More particularly, it relates to cloning vectors ensuring expression of said segment in microorganisms and to the microorganisms so transformed by said vectors. It also relates to a process for the production of said lipase by growing such transformed microorganisms.

BACKGROUND OF THE INVENTION

It is well known that the mold Geotrichum candidum produces an extracellular lipase which displays an unique, although not absolute, specificity for fatty acids comprising at least one cis-9 double bond, whatever may be the position occupied by the fatty acid on the triglyceride molecule, i.e. no specificity is observed with respect to the position of the fatty acids within the triglyceride (For a review on the specificity of the G. candidum lipase, see for example R.G. Jensen, Lipids 9, no3, 149, 1974). This specificity may be of interest for industrial purposes since it is known that the severe conditions (230-250°C, 30-40 atmospheres) applied in classical processes to hydrolyse natural fats and oils result in extensive formation of condensation and polymerisation by-products, the more so the starting natural product is richer in unsaturated fatty acids, e.g. soja, sunflower, rapeseed, cartham, linseed oil, etc...

Other practical applications of the lipase from G. candidum may result from another apparently unique property of this enzyme which, in contrast with other lipases, was shown to be able to bring about the esterification of oleic acid with secondary alcohols such as 2-propanol, 3-hexanol or cyclohexanol (S. Okumura et al., Biochim. Biophys. Acta 575, 156, 1969. Moreover, it is known that the lipase of G. candidum may find applications in the food industry, particularly as flavouring agent in the dairy industry (M.D.D. Howlett, Biotechnol. in the Food & Drink Ind., First Ntl Conf. Brighton 19-20/9/1983, Conf. Proc. p. 64).

However, these interesting features have so far never been exploited for practical purposes, except for transesterification reactions (Belgian patent no 851,265; 1977). This paradoxical situation probably results from the difficulties inherent to the production of the lipase by the use of G. candidum itself. Not only is this production hampered by the practical problems currently encountered in the large-scale growth of filamentous fungi, but the productivity of G. candidum for the lipase is generally small. As a consequence, producing the lipase necessarily requires co-producing a large amount of biomass, the valorization of which is made difficult by the fact that G. candidum is known to be responsible in men of various pathological manifestations known as geotrichosis (Dictionnaire de Médecine, Flammarion 1982, p. 342).

A way to obviate those difficulties might be to have the lipase produced by the fungus appropriately immobilized. However, this method is made unpractical in the present case by the fact that the production of lipase by G. candidum strictly requires aerobic conditions (Y. Tsujisaka et al., Agr. Biol. Chem. 37, no4,837, 1973) and the presence of lipids M. Iwai et al., Agr. Biol. Chem. 37, no 4, 929, 1973) making the medium a complex polyphasic system.

SUMMARY OF THE INVENTION

It is the main object of the present invention to make the lipase of G. candidum, or any variant thereof having retained substantially the same specificity, available for industrial applications by having it produced by a selected microorganism genetically engineered for this purpose.

This object is accomplished by providing a DNA segment coding for a lipase catalyzing the hydrolysis of triglycerides with such a specificity that fatty acids comprising at least one cis-9 double bond are selectively liberated, said segment comprising a sequence being selected in the group consisting of the following sequence and the subfragments and mutants thereof coding for lipases having retained substantially the same specificity.

```
        10        20        30        40        50        60        70        80
ATGAAATTTGTAAAAAGAAGGATCATTGCACTTGTAACAATTTTGATGCTGTCTGTTACATCGCTGTTTGCGTTGCAGCC


        90       100       110       120       130       140       150       160
GTCAGCAAAAGCCGCTGAACACAATCCAGTCGTTATGGTTCACGGTATTGGAGGGGCACCATTCAATTTTGCGGGAACTA


       170       180       190       200       210       220       230       240
AGAGCTATCCCGTATCTCAGGGCTGGTCGCGGGACAAGCTGTATGCAGTTGATTTTTGGGACAAGACAGGCACAAATTAT


       250       260       270       280       290       300       310       320
AACAATGGACCGGTATTATCGCGATTTGTGCAAAAGGTTTTAGATGAAACGGGTGCGAAAAAAGTGGATATTGTCGCTCG


       330       340       350       360       370       380       390       400
CAGCATGGGGGGCGCGAACACACTTTACTACATAAAAAATCTGGACGGCGGAAATAAAGTTGCAAACGTCGTGACGCTTG


      ·410       420       430       440       450       460       470       480
GCGGCGCGAACCGTTTGACGACAGGCAAGGCGCTTCCGGGAACAGATCCAAATCAAAAGATTTTATACACATCCATTTAC


       490       500       510       520       530       540       550       560
AGCAGTGCCGATATGATTGTCATGAATTACTTATCAAGATTAGATGGTGCTAGAAACGTTCAAATCCATGGCGTTGGACA


       570       580       590       600       610       620       630       640
CATCGGCCTTCTGTACAGCAGCCAAGTCAACAGCCTGATTAAAGAAGGGCTGAACGGCGGGGGCCAGAATACGAATTAA
```

The present invention also provides cloning vectors ensuring the expression of said segment in a microorganism selected as host, the micro-organisms thus transformed by said vectors and a fermentation process for the production of said lipase by said transformed microorganisms.

## BRIEF DESCRIPTION OF THE FIGURES

In the examples given hereinafter, reference will be made to the appended figures in which

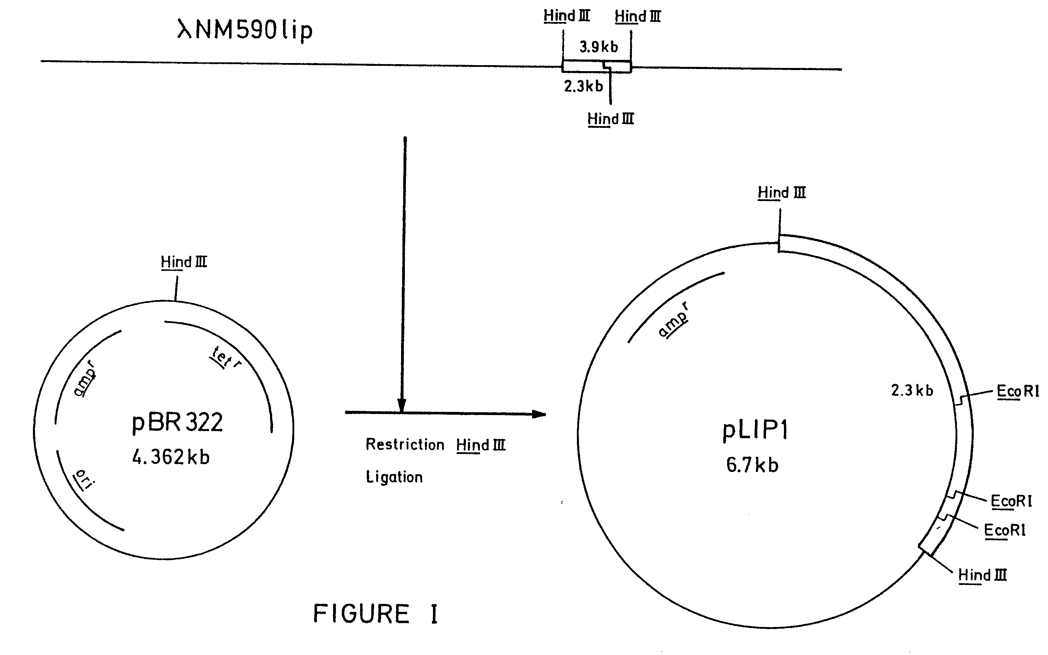

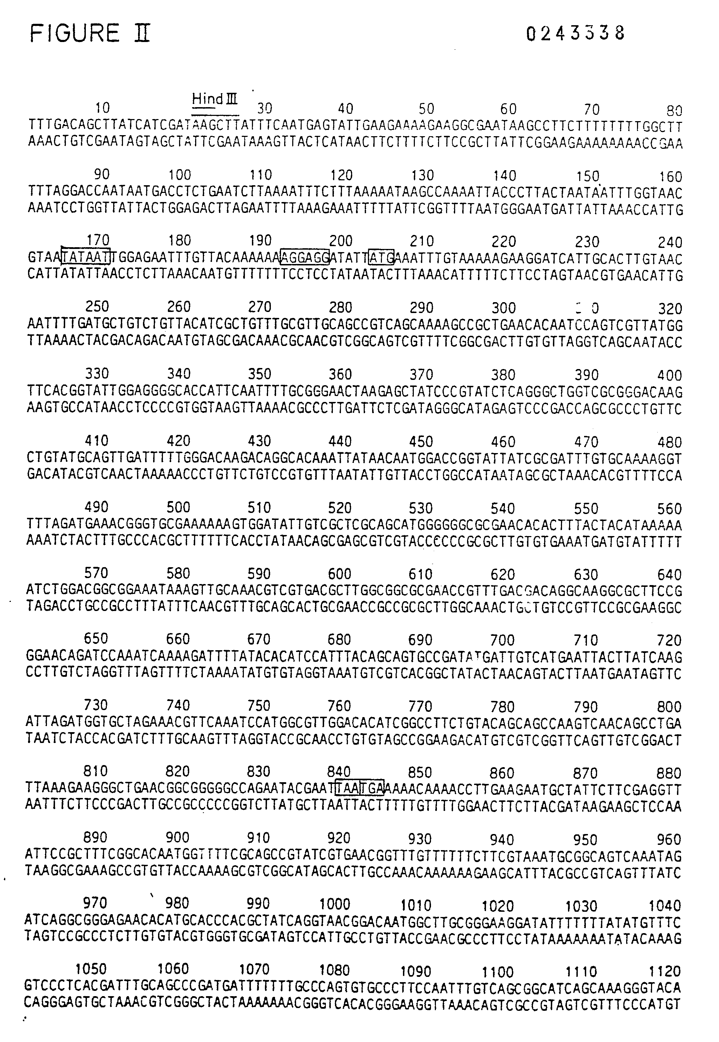

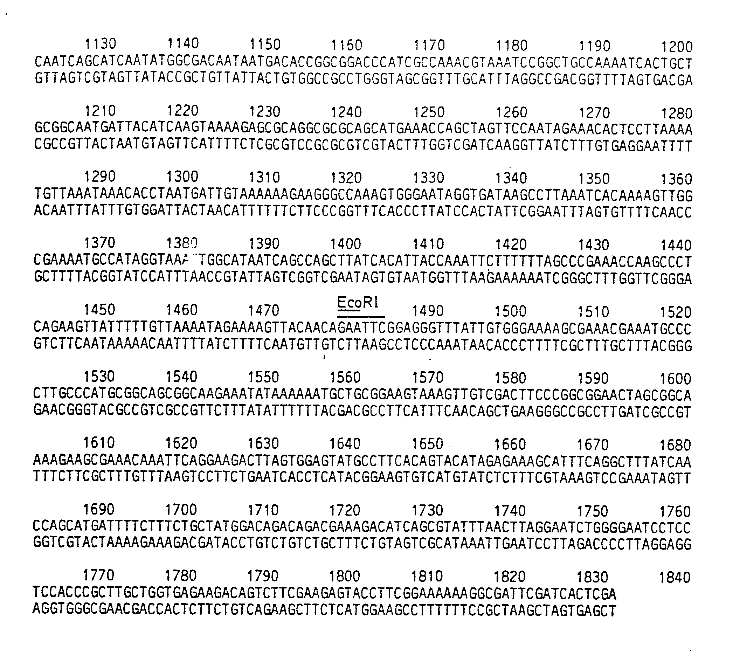

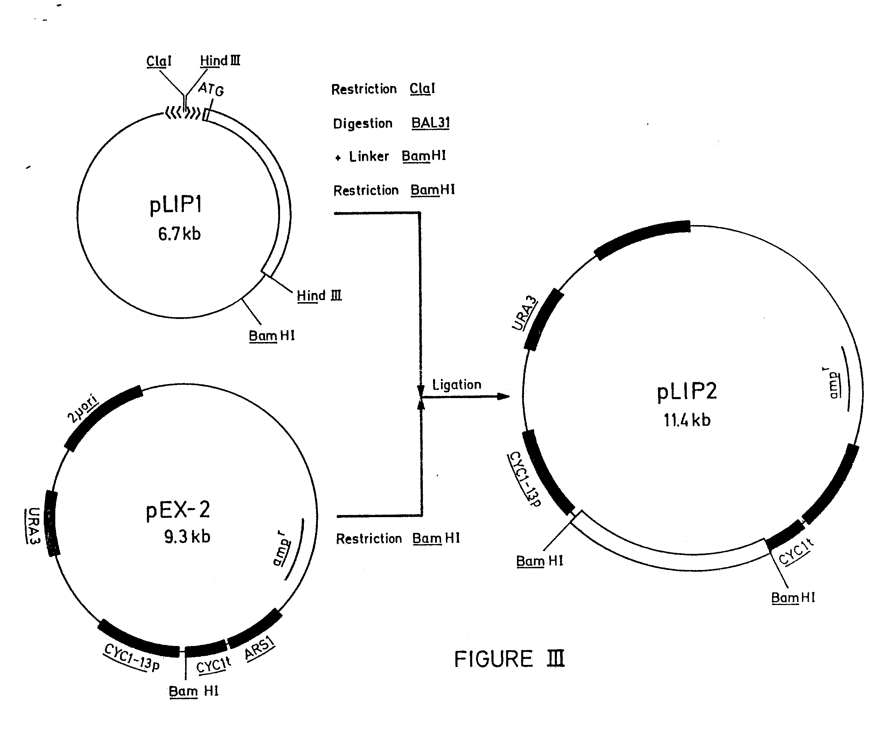

FIGURE I shows the construction of plasmid pLIP1 used to express in Escherichia coli the lipase-coding fragment of the invention. This construction was carried out by ligation of plasmid pBR322 opened at its unique HindIII restriction site with a 2.3 kb fragment isolated from the genome of the mold G.candidum by insertion in phage λNM590. On the figure, the DNA from G.candidum is represented by double lines.

FIGURE II shows the sequence of the first 1831 base pairs of a DNA region comprising the HindIII-EcoRI segment to which is associated the lipase activity displayed by E.coli transformed with plasmid pLIP1

FIGURE III shows the construction of plasmid pLIP2 used to express in yeast the lipase-coding fragment of the invention. This construction is carried out by insertion in expression vector pEX-2 of the lipase-coding fragment from plasmid pLIP1 after deletion of the region upstream from the ATG initiation codon thereof. On the figure, the DNA from G.candidum is represented by double lines, bacterial DNA by thin lines and yeast DNA by thick lines.

## DETAILED DESCRIPTION OF THE INVENTION

Apart from its specificity properties, the lipase of G. candidum has been relatively little studied so that its enzymological characteristics are still for a large part obscure. For instance its molecular weight was reported by some authors to be 37,000 (J.K. Kroll et al., Pharmazie 28 H4, 263, 1973) and by some others to be 53,000-55,000 (Y. Tsujisaka et al. Agr. Biol. Chem. 37, no 6, 1457, 1973). It appears to be slightly glycosylated so as to contain about 7.5% carbohydrates consisting mainly of mannose. It contains also a small proportion (about 1%) of lipids (Y. Tsujisaka et al., loc. cit.) but it is not known whether these play any essential role on enzyme activity. Only preliminary X-ray diffraction studies were carried out so far (Y. Hata et al., H. Biochem. 86, 1821, 1979) from which only limited information can be drawn on the enzyme secondary and tertiary structures. Its composition in amino acids was determined (Y. Tsujisaka et al, loc. cit.) : no sulphur-containing amino acids (cysteine and methionine) were detected in the hydrolysate. However, more recent determinations made from commercial preparations of purified lipase (Germe, Marseille) reveal the presence of methionine as shown in the following table wherein is also given the composition as calculated from the nucleotide sequence given hereabove.

Table. Amino acid composition of the lipase of <u>G.candidum</u> (in mole %)

| Amino acid | From Tsusijaka et al. | New determination | From nucleotide sequence |
|---|---|---|---|
| Trp | 1.8 | 1.8* | 0.9 |
| Lys | 3.8 | 4.0 | 6.6 |
| His | 2.4 | 2.6 | 1.9 |
| Arg | 3.7 | 3.9 | 3.8 |
| Asp + Asn | 13.1 | 14.8 | 12.3 |
| Thr | 5.2 | 5.5 | 6.1 |
| Ser | 6.8 | 7.7 | 7.1 |
| Glu + Gln | 7.4 | 7.7 | 4.7 |
| Pro | 6.5 | 3.0 | 3.3 |
| Gly | 9.8 | 9.5 | 11.3 |
| Ala, | 8.2 | 8.2 | 7.6 |
| Cys | 0.0 | 0.0 | 0.0 |
| Val | 6.0 | 5.2 | 9.4 |
| Met | 0.0 | 2.1 | 2.8 |
| Ile | 4.3 | 3.9 | 5.7 |
| Leu | 9.9 | 9.6 | 9.4 |
| Tyr | 4.5 | 4.8 | 4.3 |
| Phe | 6.6 | 5.7 | 2.8 |

* assuming the same value as determined by Tsujisaka et al.

The discrepancies between the composition as calculated from the nucleotide sequence given hereabove and the analytical data obtained from actual preparations of the lipase can in part be ascribed to insufficient purification of the enzyme but also to the fact that the lipase is normally secreted by G. candidum (W.O. Nelson, J. Dairy Sci. 35, 455, 1952). Therefore, the polypeptide encoded by the lipase gene should comprise a signal sequence allowing transport of the lipase into endoplasmic reticulum and which is cleaved off in the mature protein. However, as it will be shown hereinafter, homogenates prepared from E.coli wherein the lipase gene has been expressed as an intracellular protein display lipase activity with the same specificity as the mature protein secreted by G. candidum. It is to be understood that the present invention not only relates to the original gene whose nucleotide sequence is given hereabove but also to all variants obtained therefrom by mutation and/or deletion and coding for polypeptides having retained at least partially the typical specificity of the G. candidum lipase. Specifically, the DNA region coding for the mature lipase as it is actually secreted by the mold has to be considered as comprised within the scope of the present invention. Similarly, should be considered as also comprised in the invention any construction wherein said region would be fused with another DNA fragment coding for a signal sequence allowing secretion of the lipase from the microorganism selected as host. For example, when yeasts are to be used for the production of heterologous polypeptides, it is current practice to fuse the DNA region coding for this polypeptide with sequences encoding the leader region of the precursor of the yeast mating pherormone α-factor (see for example A.J. Brake et al., Proc. Natl. Acad. Sci. USA 81, 4642, 1984). Another possibility is to fuse the interesting coding region with the DNA fragment coding for an heterologous signal sequence which is recognised by the yeast cleavage mechanism, e.g. the signal sequence of chicken lysozyme (Belgian Patent no 901,223; 1985). Such constructions aiming at ensuring secretion by yeast of a polypeptide having substantially the same lipolytic activity and specificity as the G. candidum lipase have to be comprised within the scope of the present invention.

According to another aspect of the present invention, there are provided the cloning vectors for ensuring expression of the lipase-coding DNA segment as hereabove defined. These vectors have obviously to be selected in consideration of the recipient microorganism chosen. When E.coli is preferred, classical multicopy plasmids as e.g. those derived from the endogenous replicons ColE1, pMB1 and p15A, and particularly those of the pBR322 series will be used with advantage.

Other bacterial hosts of the genus Bacillus, Pseudomonas, Streptomyces, ... can also be used for cloning the lipase-coding segment. However, according to a preferred embodiment of the invention, this cloning will be carried out in yeast. As those skilled in the art know, yeasts have various practical advantages over bacteria for industrial purposes, especially for food and feed applications. Not only are several yeast species GRAS, but they are also a valuable source of proteins and vitamins for feed formulations. Moreover, they are especially suitable for large-scale fermentations, being not susceptible to phage infection and requiring only semi-sterile conditions. Another advantage of yeasts as industrial microorganisms for the production of polypeptides is that in many cases they are amenable through genetic engineering to protein excretion so that the desired polypeptide can be readily recovered from the culture medium by classical down-stream processing means such as chromatography, ultrafiltration, precipitation, etc... Still another advantage of yeasts result from the fact that they can be grown under anaerobic conditions, this making easier their use in immobilized form for the production of metabolites, including enzymes. For such purposes, the yeast Saccharomyces cerevisiae whose molecular biology is especially well known and which has been used since immemorial times in practical food applications is especially suitable. However, other species of yeast transformable by exogenous DNA can also be used. As examples of such other yeasts, one may cite Saccharomycopsis lipolytica, Schizosaccharomyces pombe, Kluyveromyces lactis, Candida cylindracea, etc... Some of these species are more prone than S. cerevisiae to excrete proteins into the culture medium. That is the case, for example, of Candida cylindracea which is well known to secrete a lipase into the culture medium from which the enzyme can be recovered, e.g. by precipitation (U.S. patent no3,189,529; 1965). Saccharomycopsis lypolytica is also known to excrete a lipase while two other lipases are secreted but remain associated with the cell wall (Y. Ota et al., Agr. Biol. Chem. 46, no 12, 2885, 1982).

However, as known by those skilled in the art, it is especially with yeasts of the genus Saccharomyces and still more especially with those strains belonging to the species S. cerevisiae that cloning techniques ensuring the production in substantial amounts of heterologous proteins are the most efficient, allowing in some cases exceptionnally high expression levels, e.g. for superoxide dismutase (International application WO 85/01503). To ensure in S. cerevisiae the expression of the lipase-coding DNA segment of the present invention, different kinds of cloning vectors are available. Integration vectors can be used for this purpose; however according to a preferred embodiment of the invention, cloning will be achieved by resorting to autonomously-replicating plasmids. Such plasmids comprise the replication origin of the 2-micron endogenous plasmid present in most strains of these species or, eventually, an ars segment of autonomous replication of chromosomial origin. Such plasmid must also contain a marker gene allowing visualization and selection of the cells which have effectively been transformed with the plasmid. As a marker gene, there is generally used a gene which codes for an enzyme involved in the biosynthesis of an essential metabolite, e.g., an amino acid. In such a case, the host cell to be used is a yeast strain which, through mutation, has become auxotrophic for this metabolite. By inoculating with this strain a medium free from said metabolite, only those cells transformed by a plasmid bearing the missing gene will be able to grow. Typical examples of such markers are the genes LEU2 and TRP1 which respectively code for an enzyme envoled in the biosynthesis of leucine and tryptophane. Another typical example is the gene URA3 which codes for an enzyme involved in the biosynthesis of uracil. These expression vectors must also comprise one, or preferably several unique restriction sites for insertion of the coding part of interest, as well as the various elements required for optimizing the expression thereof, i.e., promoters, terminators, and other control elements.

These yeast vector plasmids often further comprise bacterial sequences capable of ensuring their replication and their selection in an intermediate bacterial host, e.g. E.coli. As classical examples of such shuttle plasmids, one may cite YEp13 (J.R. Broach et al., Gene 8, 1979, 121) pFL1-4 (M.R. Chevallier et al., Gene 11, 1980, 11), pJDB207 (J.D. Beggs Alfred Benson Symposium n° 16, Munksgaard, Copenhaegen, 1981, p. 383), pHM158 and pJO158 (M. Heusterspreute et al., Gene, 34, 1985, 363-366).

According to a preferred embodiment of the invention, a plasmid comprising at least the replication (REP) functions of the sequence of the 2-micron endogenous plasmid is used, mainly when the host cell belongs to the S. cerevisiae species. Said functions generally bring to the plasmid a greater stability, particularly if the host cell has beforehand been cured of its 2-micron plasmids (C.P. Hollenberg, Curr. Top. Microbiol. Immunol. 96, 1982, 119; R.M. Walmsley et al., Mol. Gen. Genet. 1983, 361). Classical examples of such vectors are plasmids pJDB219 and pJDB248. (J.D. Beggs, Nature 275, 1978, 104).

Finally, to ensure an expression level as high as possible of the coding part of interest, it is necessary to associate it with a promoter as efficient as possible. Various strong promoters are known in yeast, e.g., the promoters of alcohol dehydrogenase (ADH1), enolase (ENO8 and ENO46), glyceraldehyde-3-phosphate dehydrogenase (GAP63 and GAP491), phosphoglycerate kinase (PGK) (M.J. Dobson et al., Nucleic Acids Res. 10, 1982, 2625), alkaline phosphatase (PH03 and PH05) (European patent application n°100,561) or still the promoter p415 or the variants thereof (Belgian patent n°901,222 1985).

These various techniques, which have been successfully applied to the cloning and the expression of many heterologous genes in yeast, can be used as well for ensuring in yeast expression of the lipase-coding segment of the present invention. Examples of specific constructions performed according to standard methodologies (T. Maniatis et al., Molecular Cloning, Cold Spring Harbor Lab., 1982) are given in the present patent for a purpose of illustration but it is evident that other possibilities exist and that various replication origins, marker genes, efficient promoters and other structural elements may be combined to obtain similar results.

In accordance with another aspect of the invention, the transformed cells obtained in those various cases must also be considered as being within the scope of the invention.

When a microorganism, bacterium or yeast, has been induced according to the present invention to produce a lipase displaying the desired specifi city for unsaturated substrates, it is necessary to grow it under the most favourable conditions to its growth in order to take advantage of this new trait. One skilled in the art will easily determine these conditions according to the characteristics peculiar to the microorganisms used as host.

As transformed microorganisms have in most cases a more or less marked tendency to lose artificially-constructed plasmids, it is advantageous to use such a culture medium as to exert a positive selection pressure on them. When the recipient yeast is a mutant auxotrophic for one or another essential metabolite and when the vector plasmid used comprises a marker gene capable of restoring prototrophy to the strain, e.g., the LEU2 or TRP1 genes mentioned above, this selection pressure may be exerted by omitting said metabolite from the culture medium. On the contrary, if the plasmid comprises as marker a gene capable of conferring to the yeast a more or less marked resistance to a growth inhibitor, e.g., an antibiotic such as G418 (J. Jimenez and J. Davies, Nature 287, 1980, 869) or an herbicide such as diuron (Belgian patent n° 899,607; 1984), the selection pressure in favor of the transformed yeast can be applied by growing it in a medium supplemented with this inhibitor. Other means exist to obtain the same result and may also be used to practice the invention.

When transformed yeasts have been grown under conditions ensuring the best production of the desired lipase, this has still to be recovered. Many techniques are available which those skilled in the art will combine to obtain in each case the best recovery yield and the greatest purity of the lipase. However, according to a preferred embodiment, the DNA region coding for said lipase will preferably be equipped with a leader sequence coding for a signal peptide capable of ensuring the transport of the lipase through the plasmic membrane of the transformed cell. In this case, the separation of the enzyme will indeed be considerably easier, whether it is liberated into the medium from where it will be recovered by classical methods or it remains associated to the yeast cell wall from which it will have to be separated by other methods.

The various aspects of the present invention will appear more specifically in the following examples which are purely illustrative and should not be construed to limit the scope of the invention.

## EXAMPLES

1. Cloning in E.coli of the lipase-coding segment, using bacteriophage λNM590 as insertion vector

A DNA preparation from phage λNM590 (N.E. Murray et al., Mol. Gen. Genet. 150, 53, 1977) and another DNA preparation from the mold G.candidum were both cleaved with restriction endonuclease HindIII. Phage λNM590 has an unique HindIII restriction site and the two fragments generated therefrom were ligated, using T4 DNA ligase, with an equivalent amount of DNA fragments from G.candidum. The recombinant DNA thus obtained was then packaged by mixing with proteins prepared by the method of B. Hohn and K. Murray (Proc. Natl. Acad. Sci. 74, n°8, 3259, 1977) from the defective phages present in the two E.coli strains BH2671 and BH2673. Complete phage particles thus obtained were then used to infect E.coli strain HB101.

Seven Petri dishes containing "Spirit Blue Agar" (Difco) and "lipase reagent" (Difco) were inoculated with infected bacteria and incubated overnight at 37°C whereby approximately 1000 plaques of lyse appeared per Petri dish.

One of these plaques showed lipase activity as evidenced by the development of a typical blue coloration. The phage responsible for this activity and called λNM590lip was purified by dilution and prepared in relatively large amount by lysing in liquid medium E.coli strain CL1204 (C600 leu serB thi lac hsdR) known to be easily infected by λphages. The phages were then separated and further purified by ultracentrifugation on preformed CsCl gradient and dialysis against Tris buffer pH 7.5.

The DNA of the phages thus purified was separated by double phenolisation and dialysis against Tris/EDTA buffer pH 8. Upon characterization by restriction analysis, it was determined that the lipase-coding region was associated with a 3.9 kb HindIII insert comprising itself two HindIII subfragments of 2.3 and 1.6 kb respectively.

## 2. Cloning in E.coli of the lipase-coding segment, using plasmid pBR322 as vector

The fragments obtained by incomplete digestion with restriction enzyme HindIII of a DNA preparation from phageλMN590lip obtained as described in the preceding example was ligated with pBR322 opened with the same enzyme and dephosphorylated by alkaline phosphatase. The recombinant DNA thus obtained was used to transform E.coli strain HB101 (leu pro lac thi rpsL recA hsdS; H.W. Boyer & D. Roulland-Dussoix, J. Mol. Biol. 41, 459, 1969) made competent by $CaCl_2$ treatment and the transformants were then screened for lipase activity by transfer on Petri dishes of "Sprit blue Agar" with "lipase reagent" : 8 clones were shown to be positive by the development of a blue halo around the colonies.

One of these positive clones was chosen for efficient lipase production and was shown to be tetracycline sensitive. This clone will be referred to hereinafter as pLIP1. The plasmid contained therein was characterized by restriction analysis followed by electrophoresis on agarose gel and was shown to contain a 2.3 kb insert. Through further characterization by electrophoresis on acrylamide gels, it was determined that this insert comprises three EcoRI sites giving rise through restriction to one relatively large HindIII-EcoRI fragment of about 1400 bp and three smaller fragments of about 520, 230 and 120 bp as shown on Figure I.

In another construction, the large and the small external HindIII-EcoRI fragments were separated and ligated through a triple ligation event with plasmid pBR322 open with restriction enzyme Hind III and the resultant recombinant plasmid used to transform HB101. The transformants thus obtained displayed lipase activity which shows that the lipase-coding region is entirely comprised within the larger HindIII-EcoRI fragment. Figure II shows the nucleotide sequence (as determined by the method of A. Maxam and W. Gilbert, Methods in Enzymology, Ed. by L. Grossman and K. Moldave, Acad. Press. inc., New York & London, Vol. 65, p.499, 1980) of a DNA segment comprising said larger fragment. It can be seen that it contains only one open reading frame coding for a protein of more than 200 amino acids : this coding region starts with an ATG initiation codon on position 203 and ends after position 838 by a double stop signal (TAA, TGA).

Moreover, the 5' flanking region thereof comprises a typical Pribnow box TATAAT 38 bp upstream from the ATG and a typical Shine-Dalgarno sequence AGGAGG starting 11 bp upstream from the ATG.

Further evidence that this coding region actually corresponds to the lipase produced by the bacteria transformed with pLIP1 plasmid was gained by the following deletion experiments : (a) plasmid pLIP1 was cleaved at its unique ClaI and NcoI sites to generate a 5800 bp fragment and a 733 bp fragment comprising the 5' end of the coding region containing itself two HpaII restriction sites; (b) the smaller fragment was cleaved with HpaII; (c) the ClaI-HpaII (434 bp) and the HpaII-NcoI (110 bp) fragments generated by the latter restriction and the 5800 bp ClaI-NcoI fragment generated by the former were purified and simultaneously ligated to give a plasmid deleted from a 186 bp HpaII-HpaII fragment within the coding region; (d) this plasmid was used to transform E.coli. No lipase activity was detected in the resulting transformants.

Finally, to ascertain that the lipase produced by the bacteria transformed with plasmid pLIP1 has the desired specificity displayed by the lipase from G.candidum, a crude enzyme preparation obtained by sonication and lyophylisation of transformed bacteria was used to hydrolyse an equimolecular mixture of triolein and tripalmitin according to the method of Cl. Franzke et al. (Die Nahrung 17, no2, 171, 1973). The free acids liberated by action of the lipase were separated by thin-layer chromatography using as solvent a mixture petroleum ether/diethylether/acetic acid 70/30/2, transformed into methyl esters by the method of P.A. Biondi & M. Cagnasso (J. Chromatography 109, 389, 1975) and analyzed by vapour-phase chromatography using a 10% Silar 5CP/chromosorb WHP 80/100 mesh (Chromopack) column. The proportion of free acids thus determined was in mole % : palmitic acid, 8%; oleic acid, 92%.

Strain E.coli HB101(pLIP1) has been deposited on April 22, 1986 at the Centraal Bureau voor Schimmelcultures, Oosterstraat 1, P.O. Box 273, NL - 3740 AG Baarn (The Netherlands), where it has been given accession number C.B.S. 229.86.

## 3. Cloning of the lipase-coding segment in yeast

### 3.1. Insertion of the 2.3 kb HindIII fragment in vector pJDB207

The 2.3 kb HindIII DNA fragment responsible for lipase activity of bacteria transformed with plasmid pLIP1 as described in the preceding example was inserted in a HindIII site of the yeast-E.coli shuttle plasmid pJDB207 (J.D. Beggs, Genetic Engineering 2, Ed. by R. Williamson, Academic Press 1981, p. 175) containing the bacterial ampr and tetr genes imparting resistance to ampicillin and tetracycline for selection in E.coli as well as the yeast LEU2 gene for selection in Leu– strains of yeast. The resulting recombinant plasmids were then used to transform E.coli strain HB101 and the transformants were screened for ampicillin resistance. Upon transfer of 100 resistant clones on "Spirit blue Agar", 9 of them were shown to be lipase-positive; they were also shown to be tetracycline sensitive. One of these clones called pEK8 was selected for transformation into yeast. Plasmid DNA from this clone was isolated by the method of H.C. Birnboim & J. Doly (Nucleic Acid Res.7, no6, 1513, 1979) and used to transform yeast strain AH22 (leu 2-3 leu 2-112 his 4-519 CAN).

Transformants were screened for leucine prototrophy on minimal medium (0.67% yeast nitrogen base, 2% glucose) supplemented with 0.002% histidine, 1M sorbitol and 2% agar. The resulting transformants were then tested for lipase activity by culturing in liquid minimal medium supplemented with histidine, disrupting the cells in phosphate buffer 0.1M pH 7 by shaking with glass beads in a Braun homogeneizer, and determining lipase activity by a radioassay adapted from the method of M.C. Scholtz et al. (J. Lip. Res. 11, 68, 1970). No significant lipase activity could be detected in the cell homogenate. To ascertain that the lipase-coding segment was actually present in the transformed yeasts, the plasmid DNA present therein was extracted and used again to transform E.coli strain HB101. The transformed clones were shown to be lipase-positive. The conclusion was that the control elements accompanying the lipase-coding region in the original 2.3 kb fragment from the G.candidum genome are correctly recognized by E.coli but not by the yeast expression machinery.

### 3.2. Cloning of the lipase-coding segment in S.cerevisiae, using expression vector pEX-2

In order to ensure expression in yeast of the lipase-coding segment of the present invention, it appeared necessary, in view of the preceding results, to delete the 5′ flanking region thereof, upstream from the ATG initiation codon, and then to fuse in an appropriate yeast expression vector the trimmed fragment with a yeast promoter. In this construction, the yeast-E.coli shuttle plasmid pEX-2 was selected as expression vector for its ability to ensure expression of heterologous genes as well in E.coli as in yeast (J.F. Ernst and R.K. Chan, J. Bacteriol. 163, no1, 8, 1985). This plasmid comprises the ampr marker gene for selection in E.coli and the URA3 marker gene for selection in Ura– strains of yeasts. The different steps of the construction were the following (see Fig. III) :

a) Plasmid pLIP1 was opened at its unique ClaI site and digested at 20ºC for 1.30 min. with nuclease BAL31 (0.1 units/µg of DNA), the latter reaction being stopped by phenolisation.

b) The population of shortened plasmids thus obtained were then recirculized using a linker BamHI 12 MER inserted by the method of R. Lathe et al. (DNA 3, 173, 1984) and transformed into E.coli strain BJ5183 (F–recBC sbcB endoI gal met str thi bio hsdR; M.R. Chevallier, Mol. & Cell. Biol. 2 no8, 977, 1982) remarkable for its high transformation frequency.

c) From 960 transformed clones, 613 (64%) had lost lipase activity. These inactive clones were pooled for growth in selective medium (1% tryptone, 1% NaCl, 0.5% yeast extract, 50 mg/l ampicillin) and isolation of plasmid DNA.

d) The plasmid DNA thus prepared was cleaved with restriction enzyme BamHI and the resulting fragments separated by electrophoresis on agarose gel. Those fragments bearing the lipase-coding region migrated as a single large band which was recovered from the gel on DEAE-cellulose paper by the method of G. Dretzen et al. (Anal. Biochem. 112, 295, 1981).

e) The fragments thus isolated were then ligated with plasmid pEX-2 opened at its unique BamHI site between the CYC1 promoter and terminator regions and dephosphorylated by action of bacterial alcaline phosphatase (Worthington).

f) The population of recombinant plasmids thus obtained were transformed into E.coli strain BJ5183 and transformants simultaneously screened for ampicillin resistance and lipase activity : 26 clones out of about 25000 were selected for their ability to develop lipase activity within 2 days as evidenced by the blue color developed on "Sprit blue Agar" supplemented with "lipase reagent" and with ampicillin for selection of transformants.

g) The plasmid DNA from these lipase-positive clones was isolated by the method of Birnboim & Doly (ref. cit.), and used to transform E.coli strain HB101 more appropriate than strain BJ5183 for DNA preparation.

The resulting transformants were then characterized by plasmid DNA isolation, BamHI restriction and agarose gel electrophoresis. One of these clones was selected for good lipase activity and for the presence of a well defined BamHI-BamHI insert of the expected size in plasmid pEX-2. This clone referred to hereinafter as pLIP2 was chosen for transformation into yeast. The nucleotide sequence of the junction between plasmid pEX-2 and the lipase-coding region was determined by the Maxam and Gilbert method and shown to be

```
.....GGATCCGGAGGATATTATGAAATTT...

  BamHI          lipase-coding region
```

h) Plasmid pLIP2 was used to transform the Ura3– yeast strain 01904B and the transformants screened on minimal medium. One of the resulting clones was cultured at 28°C in liquid minimal medium till stationnary phase (about 4 days). The cells were then harvested by centrifugation, resuspended in a phosphate buffer 0.1M pH7 and disrupted by shaking with glass beads in a Braun homogeneizer. A 0.2 ml part of the resulting homogenate was used to determine lipase activity by the same radioassay as used above, taking the lipase commercialized by Germe (Marseille, France) as a standard. Total proteins were determined on another aliquot part by the method of Lowry modified by D. Herbert et al. (P.R. Stewart, Methods in Cell Biol., Ed. by P.R. Stewart Vol. XII, p. 113, 1981). It was thus determined that 17 international units of lipase (1 unit = amount of lipase bringing about the liberation of 1 mg of fatty acids per minute) corresponding to 0.342 mg of enzyme (specific activity : about 300 u/mg) were present in 20.5 mg of total proteins, i.e. the expressed lipase corresponded to 1.7% of total proteins.

**Claims**

1) DNA segment coding for a lipase which catalyses the hydrolysis of triglycerides with a specificity for fatty acids containing cis-9 unsaturation, characterized in that said segment comprises a sequence selected from the group comprising the following nucleotide sequence :

```
          10        20        30        40        50        60        70        80
ATGAAATTTGTAAAAAGAAGGATCATTGCACTTGTAACAATTTTGATGCTGTCTGTTACATCGCTGTTTGCGTTGCAGCC


          90       100       110       120       130       140       150       160
GTCAGCAAAAGCCGCTGAACACAATCCAGTCGTTATGGTTCACGGTATTGGAGGGGCACCATTCAATTTTGCGGGAACTA


         170       180       190       200       210       220       230       240
AGAGCTATCCCGTATCTCAGGGCTGGTCGCGGGACAAGCTGTATGCAGTTGATTTTTGGGACAAGACAGGCACAAATTAT


         250       260       270       280       290       300       310       320
AACAATGGACCGGTATTATCGCGATTTGTGCAAAAGGTTTTAGATGAAACGGGTGCGAAAAAAGTGGATATTGTCGCTCG


         330       340       350       360       370       380       390       400
CAGCATGGGGGGCGCGAACACACTTTACTACATAAAAAAATCTGGACGGCGGAAATAAAGTTGCAAACGTCGTGACGCTTG


         410       420       430       440       450       460       470       480
GCGGCGCGAACCGTTTGACGACAGGCAAGGCGCTTCCGGGAACAGATCCAAATCAAAAGATTTTATACACATCCATTTAC


         490       500       510       520       530       540       550       560
AGCAGTGCCGATATGATTGTCATGAATTACTTATCAAGATTAGATGGTGCTAGAAACGTTCAAATCCATGGCGTTGGACA


         570       580       590       600       610       620       630       640
CATCGGCCTTCTGTACAGCAGCCAAGTCAACAGCCTGATTAAAGAAGGGCTGAACGGCGGGGGCCAGAATACGAATTAA
```

and subfragments and mutants thereof coding for lipases having retained substantially the same specificity.

2. Cloning vector capable of ensuring in a yeast the expression of a DNA segment coding for a lipase which catalyses the hydrolysis of triglycerides with a specificity for fatty acids containing cis-9 unsaturation, characterized in that said DNA segment is selected from the group comprising the following nucleotide sequence

ATGAAATTTGTAAAAAGAAGGATCATTGCACTTGTAACAATTTTGATGCTGTCTGTTACATCGCTGTTTGCGTTGCAGCC

GTCAGCAAAAGCCGCTGAACACAATCCAGTCGTTATGGTTCACGGTATTGGAGGGGCACCATTCAATTTTGCGGGAACTA

AGAGCTATCCCGTATCTCAGGGCTGGTCGCGGGACAAGCTGTATGCAGTTGATTTTTGGGACAAGACAGGCACAAATTAT

AACAATGGACCGGTATTATCGCGATTTGTGCAAAAGGTTTTAGATGAAACGGGTGCGAAAAAAGTGGATATTGTCGCTCG

CAGCATGGGGGGCGCGAACACACTTTACTACATAAAAAATCTGGACGGCGGAAATAAAGTTGCAAACGTCGTGACGCTTG

GCGGCGCGAACCGTTTGACGACAGGCAAGGCGCTTCCGGGAACAGATCCAAATCAAAAGATTTTATACACATCCATTTAC

AGCAGTGCCGATATGATTGTCATGAATTACTTATCAAGATTAGATGGTGCTAGAAACGTTCAAATCCATGGCGTTGGACA

CATCGGCCTTCTGTACAGCAGCCAAGTCAACAGCCTGATTAAAGAAGGGCTGAACGGCGGGGGCCAGAATACGAATTAA

and subfragments and mutants thereof coding for lipases which have retained substantially the same specificity.

3. Cloning vector according to claim 2 characterized in that the DNA segment coding for the lipase is fused with a DNA segment coding for a signal sequence allowing the secretion of the lipase from said yeast.

4. Cloning vector according to claim 3, characterized in that the signal sequence is selected from the group comprising the signal sequence of invertase, acid phosphatase, α-mating factor, a-mating factor, chicken lysozyme, Escherichia coli beta-lactamase and Aspergillus awamori glucoamylase.

5. Cloning vectors capable of ensuring in a bacterium the expression of a DNA segment coding for a lipase which catalyses the hydrolysis of triglycerides with a specificity for fatty acids containing cis-9 unsaturation, characterized in that said DNA segment is selected from the group comprising the following nucleotide sequence

```
         10        20        30        40        50        60        70        80
ATGAAATTTGTAAAAAGAAGGATCATTGCACTTGTAACAATTTTGATGCTGTCTGTTACATCGCTGTTTGCGTTGCAGCC

         90       100       110       120       130       140       150       160
GTCAGCAAAAGCCGCTGAACACAATCCAGTCGTTATGGTTCACGGTATTGGAGGGGCACCATTCAATTTTGCGGGAACTA

        170       180       190       200       210       220       230       240
AGAGCTATCCCGTATCTCAGGGCTGGTCGCGGGACAAGCTGTATGCAGTTGATTTTTGGGACAAGACAGGCACAAATTAT

        250       260       270       280       290       300       310       320
AACAATGGACCGGTATTATCGCGATTTGTGCAAAAGGTTTTAGATGAAACGGGTGCGAAAAAAGTGGATATTGTCGCTCG

        330       340       350       360       370       380       390       400
CAGCATGGGGGGCGCGAACACACTTTACTACATAAAAAAATCTGGACGGCGGAAATAAAGTTGCAAACGTCGTGACGCTTG

        410       420       430       440       450       460       470       480
GCGGCGCGAACCGTTTGACGACAGGCAAGGCGCTTCCGGGAACAGATCCAAATCAAAAGATTTTATACACATCCATTTAC

        490       500       510       520       530       540       550       560
AGCAGTGCCGATATGATTGTCATGAATTACTTATCAAGATTAGATGGTGCTAGAAACGTTCAAATCCATGGCGTTGGACA

        570       580       590       600       610       620       630       640
CATCGGCCTTCTGTACAGCAGCCAAGTCAACAGCCTGATTAAAGAAGGGCTGAACGGCGGGGGCCAGAATACGAATTAA
```

and the subfragments and mutants thereof coding for lipases which have retained substantially the same specificity.

6. Cloning vector according to claim 5, characterized in that the DNA segment coding for the lipase is fused with a DNA segment coding for a signal sequence allowing the secretion of the lipase from said bacterium.

7. Vector plasmid capable of ensuring in a yeast the expression of a DNA segment coding for a lipase which catalyses the hydrolysis of triglycerides with a specificity for fatty acids containing a cis-9 unsaturation, characterized in that said DNA segment is selected from the group comprising the following nucleotide sequence

```
          10        20        30        40        50        60        70        80
ATGAAATTTGTAAAAAGAAGGATCATTGCACTTGTAACAATTTTGATGCTGTCTGTTACATCGCTGTTTGCGTTGCAGCC

          90       100       110       120       130       140       150       160
GTCAGCAAAAGCCGCTGAACACAATCCAGTCGTTATGGTTCACGGTATTGGAGGGGCACCATTCAATTTTGCGGGAACTA

         170       180       190       200       210       220       230       240
AGAGCTATCCCGTATCTCAGGGCTGGTCGCGGGACAAGCTGTATGCAGTTGATTTTTGGGACAAGACAGGCACAAATTAT

         250       260       270       280       290       300       310       320
AACAATGGACCGGTATTATCGCGATTTGTGCAAAAGGTTTTAGATGAAACGGGTGCGAAAAAAGTGGATATTGTCGCTCG

         330       340       350       360       370       380       390       400
CAGCATGGGGGGCGCGAACACACTTTACTACATAAAAAATCTGGACGGCGGAAATAAAGTTGCAAACGTCGTGACGCTTG

         410       420       430       440       450       460       470       480
GCGGCGCGAACCGTTTGACGACAGGCAAGGCGCTTCCGGGAACAGATCCAAATCAAAAGATTTTATACACATCCATTTAC

         490       500       510       520       530       540       550       560
AGCAGTGCCGATATGATTGTCATGAATTACTTATCAAGATTAGATGGTGCTAGAAACGTTCAAATCCATGGCGTTGGACA

         570       580       590       600       610       620       630       640
CATCGGCCTTCTGTACAGCAGCCAAGTCAACAGCCTGATTAAAGAAGGGCTGAACGGCGGGGGCCAGAATACGAATTAA
```

and the subfragments and mutants thereof coding for lipases which have retained substantially the same specificity.

8. Vector plasmid according to claim 7 characterized in that the DNA segment coding for the lipase is fused with a DNA segment coding for a signal sequence allowing the secretion of the lipase from said yeast.

9. Vector plasmid according to claim 8 characterized in that the signal sequence is selected from the group comprising the signal sequence of invertase, acid phosphatase, α-mating factor, a-mating factor, chicken lysozyme, Escherichia coli beta-lactamase and Aspergillus awamori glucoamylase.

10. Vector plasmid capable of ensuring in a bacterium the expression of a DNA segment coding for a lipase which catalyses the hydrolysis of triglycerides with a specificity for fatty acid containing a cis-9 unsaturation, characterized in that said DNA segment is selected from the group comprising the following nucleotide sequence

```
        10        20        30        40        50        60        70        80
ATGAAATTTGTAAAAAGAAGGATCATTGCACTTGTAACAATTTTGATGCTGTCTGTTACATCGCTGTTTGCGTTGCAGCC


        90       100       110       120       130       140       150       160
GTCAGCAAAAGCCGCTGAACACAATCCAGTCGTTATGGTTCACGGTATTGGAGGGGCACCATTCAATTTTGCGGGAACTA


       170       180       190       200       210       220       230       240
AGAGCTATCCCGTATCTCAGGGCTGGTCGCGGGACAAGCTGTATGCAGTTGATTTTTGGGACAAGACAGGCACAAATTAT


       250       260       270       280       290       300       310       320
AACAATGGACCGGTATTATCGCGATTTGTGCAAAAGGTTTTAGATGAAACGGGTGCGAAAAAAGTGGATATTGTCGCTCG


       330       340       350       360       370       380       390       400
CAGCATGGGGGGCGCGAACACACTTTACTACATAAAAAATCTGGACGGCGGAAATAAAGTTGCAAACGTCGTGACGCTTG


       ·410       420       430       440       450       460       470       480
GCGGCGCGAACCGTTTGACGACAGGCAAGGCGCTTCCGGGAACAGATCCAAATCAAAAGATTTTATACACATCCATTTAC


       490       500       510       520       530       540       550       560
AGCAGTGCCGATATGATTGTCATGAATTACTTATCAAGATTAGATGGTGCTAGAAACGTTCAAATCCATGGCGTTGGACA


       570       580       590       600       610       620       630       640
CATCGGCCTTCTGTACAGCAGCCAAGTCAACAGCCTGATTAAAGAAGGGCTGAACGGCGGGGGCCAGAATACGAATTAA
```

and the subfragments and mutants thereof coding for lipases which have retained substantially the same specificity.

11. Plasmid according to claim 10 characterized in that the DNA segment coding for the lipase is fused with a DNA segment coding for a signal sequence allowing the secretion of the lipase from said bacterium.

12. Vector plasmid capable of autonomous replication in a yeast and capable of ensuring in said yeast the expression of a DNA segment coding for a lipase which catalyses the hydrolysis of triglycerides with a specificity for fatty acids containing a cis-9 unsaturation, characterized in that said DNA segment is selected from the group comprising the following nucleotide sequence

0 243 338

```
          10      20      30      40      50      60      70      80
ATGAAATTTGTAAAAAGAAGGATCATTGCACTTGTAACAATTTTGATGCTGTCTGTTACATCGCTGTTTGCGTTGCAGCC

          90     100     110     120     130     140     150     160
GTCAGCAAAAGCCGCTGAACACAATCCAGTCGTTATGGTTCACGGTATTGGAGGGGCACCATTCAATTTTGCGGGAACTA

         170     180     190     200     210     220     230     240
AGAGCTATCCCGTATCTCAGGGCTGGTCGCGGGACAAGCTGTATGCAGTTGATTTTTGGGACAAGACAGGCACAAATTAT

         250     260     270     280     290     300     310     320
AACAATGGACCGGTATTATCGCGATTTGTGCAAAAGGTTTTAGATGAAACGGGTGCGAAAAAAGTGGATATTGTCGCTCG

         330     340     350     360     370     380     390     400
CAGCATGGGGGGCGCGAACACACTTTACTACATAAAAAATCTGGACGGCGGAAATAAAGTTGCAAACGTCGTGACGCTTG

         410     420     430     440     450     460     470     480
GCGGCGCGAACCGTTTGACGACAGGCAAGGCGCTTCCGGGAACAGATCCAAATCAAAAGATTTTATACACATCCATTTAC

         490     500     510     520     530     540     550     560
AGCAGTGCCGATATGATTGTCATGAATTACTTATCAAGATTAGATGGTGCTAGAAACGTTCAAATCCATGGCGTTGGACA

         570     580     590     600     610     620     630     640
CATCGGCCTTCTGTACAGCAGCCAAGTCAACAGCCTGATTAAAGAAGGGCTGAACGGCGGGGGCCAGAATACGAATTAA
```

and the subfragments and mutants thereof coding for lipases which have retained substantially the same specificity.

13. Vector plasmid according to claim 12 characterized in that its replication is ensured by the presence of sequences from the 2-micron plasmid and comprising at least the replication origin of the 2-micron plasmid.

14. Vector plasmid according to anyone of claims 12 and 13 characterized in that the DNA segment coding for the lipase is fused with a DNA segment coding for a signal sequence allowing the secretion of the lipase from said yeast.

15. Vector plasmid according to claim 14 characterized in that the signal sequence is selected from the group comprising the signal sequence of invertase, acid phosphatase, α-mating factor, a-mating factor, chicken lysozyme, Escherichia coli beta-lactamase and Aspergillus awamori gluco-amylase.

16. Vector plasmid according to anyone of claims 12 and 13, characterized in that said plasmid is plasmid pLIP2.

17. Vector plasmid capable of autonomous replication in a bacterium and capable of ensuring in said bacterium the expression of a DNA segment coding for a lipase which catalyses the hydrolysis of triglycerides with a specificity for fatty acids containing a cis-9 unsaturation, characterized in that said DNA segment is selected from the group comprising the following nucleotide sequence

14

```
        10        20        30        40        50        60        70        80
ATGAAATTTGTAAAAAGAAGGATCATTGCACTTGTAACAATTTTGATGCTGTCTGTTACATCGCTGTTTGCGTTGCAGCC

        90       100       110       120       130       140       150       160
GTCAGCAAAAGCCGCTGAACACAATCCAGTCGTTATGGTTCACGGTATTGGAGGGGCACCATTCAATTTTGCGGGAACTA

       170       180       190       200       210       220       230       240
AGAGCTATCCCGTATCTCAGGGCTGGTCGCGGGACAAGCTGTATGCAGTTGATTTTTGGGACAAGACAGGCACAAATTAT

       250       260       270       280       290       300       310       320
AACAATGGACCGGTATTATCGCGATTTGTGCAAAAGGTTTTAGATGAAACGGGTGCGAAAAAAGTGGATATTGTCGCTCG

       330       340       350       360       370       380       390       400
CAGCATGGGGGGCGCGAACACACTTTACTACATAAAAAAATCTGGACGGCGGAAATAAAGTTGCAAACGTCGTGACGCTTG

       410       420       430       440       450       460       470       480
GCGGCGCGAACCGTTTGACGACAGGCAAGGCGCTTCCGGGAACAGATCCAAATCAAAAGATTTTATACACATCCATTTAC

       490       500       510       520       530       540       550       560
AGCAGTGCCGATATGATTGTCATGAATTACTTATCAAGATTAGATGGTGCTAGAAACGTTCAAATCCATGGCGTTGGACA

       570       580       590       600       610       620       630       640
CATCGGCCTTCTGTACAGCAGCCAAGTCAACAGCCTGATTAAAGAAGGGCTGAACGGCGGGGGCCAGAATACGAATTAA
```

and the subfragments and mutants thereof coding for lipases which have retained substantially the same specificity.

18. Vector plasmid according to claim 17, characterized in that the DNA segment coding for the lipase is fused with a DNA segment coding for a signal sequence allowing secretion of the lipase from said bacterium.

19. Vector plasmid according to claim 17, characterized in that said plasmid is plasmid pLIP1.

20. Transformed yeast characterized in that it comprises a plasmid according to anyone of claims 7, 8, 9, 12, 13, 14, 15 and 16.

21. Transformed yeast according to claim 20 characterized in that it belongs to the genus selected from the group comprising Saccharomyces, Kluyveromyces, Schizosaccharomyces, Saccharomycopsis and Candida

22. Transformed yeast accordig to claim 21, characterized in that it belongs to the species selected from the group comprising Saccharomyces cerevisiae, Saccharomycopsis lipolytica, Schizosaccharomyces pombe, Kluyveromyces lactis, Candida cylindracea.

23. Transformed bacterium characterized in that it comprises a plasmid according to anyone of claims 10, 11, 17, 18 and 19.

24. Transformed bacterium according to claim 23, characterized in that said bacterium is selected from the group comprising Escherichia coli and Bacillus subtilis.

25. Process for preparing a lipase which catalyses the hydrolysis of triglycerides with a specificity for fatty acids containing cis-9 unsaturation, characterized in that it consists of growing a yeast according to claims 20, 21 and 22 and recovering the lipase thus produced.

26. Process for preparing a lipase which catalyses the hydrolysis of triglycerides with a specificity for fatty acids containing cis-9 unsaturation, characterized in that it consists of growing a bacterium according to claims 23 and 24 and recovering the lipase thus produced.

27. Lipase obtained according to anyone of claims 25 and 26.

FIGURE I

FIGURE II                                    0243338

```
                       Hind III
     10              20        30        40        50        60        70      80
TTTGACAGCTTATCATCGATAAGCTTATTTCAATGAGTATTGAAGAAAAGAAGGCGAATAAGCCTTCTTTTTTTTGGCTT
AAACTGTCGAATAGTAGCTATTCGAATAAAGTTACTCATAACTTCTTTTCTTCCGCTTATTCGGAAGAAAAAAAACCGAA

     90       100       110       120       130       140       150      160
TTTAGGACCAATAATGACCTCTGAATCTTAAAATTTCTTTAAAAATAAGCCAAAATTACCCTTACTAATAATTTGGTAAC
AAATCCTGGTTATTACTGGAGACTTAGAATTTTAAAGAAATTTTTATTCGGTTTTAATGGGAATGATTATTAAACCATTG

     170       180       190       200       210       220       230      240
GTAATATAATTGGAGAATTTGTTACAAAAAAAGGAGGATATTATGAAATTTGTAAAAAGAAGGATCATTGCACTTGTAAC
CATTATATTAACCTCTTAAACAATGTTTTTTTCCTCCTATAATACTTTAAACATTTTTCTTCCTAGTAACGTGAACATTG

     250       260       270       280       290       300       310      320
AATTTTGATGCTGTCTGTTACATCGCTGTTTGCGTTGCAGCCGTCAGCAAAAGCCGCTGAACACAATCCAGTCGTTATGG
TTAAAACTACGACAGACAATGTAGCGACAAACGCAACGTCGGCAGTCGTTTTCGGCGACTTGTGTTAGGTCAGCAATACC

     330       340       350       360       370       380       390      400
TTCACGGTATTGGAGGGGCACCATTCAATTTTGCGGGAACTAAGAGCTATCCCGTATCTCAGGGCTGGTCGCGGGACAAG
AAGTGCCATAACCTCCCCGTGGTAAGTTAAAACGCCCTTGATTCTCGATAGGGCATAGAGTCCCGACCAGCGCCCTGTTC

     410       420       430       440       450       460       470      480
CTGTATGCAGTTGATTTTTGGGACAAGACAGGCACAAATTATAACAATGGACCGGTATTATCGCGATTTGTGCAAAAGGT
GACATACGTCAACTAAAAACCCTGTTCTGTCCGTGTTTAATATTGTTACCTGGCCATAATAGCGCTAAACACGTTTTCCA

     490       500       510       520       530       540       550      560
TTTAGATGAAACGGGTGCGAAAAAAGTGGATATTGTCGCTCGCAGCATGGGGGGCGCGAACACACTTTACTACATAAAAA
AAATCTACTTTGCCCACGCTTTTTTCACCTATAACAGCGAGCGTCGTACCCCCCGCGCTTGTGTGAAATGATGTATTTTT

     570       580       590       600       610       620       630      640
ATCTGGACGGCGGAAATAAAGTTGCAAACGTCGTGACGCTTGGCGGCGCGAACCGTTTGACGACAGGCAAGGCGCTTCCG
TAGACCTGCCGCCTTTATTTCAACGTTTGCAGCACTGCGAACCGCCGCGCTTGGCAAACTGCTGTCCGTTCCGCGAAGGC

     650       660       670       680       690       700       710      720
GGAACAGATCCAAATCAAAAGATTTTATACACATCCATTTACAGCAGTGCCGATATGATTGTCATGAATTACTTATCAAG
CCTTGTCTAGGTTTAGTTTTCTAAAATATGTGTAGGTAAATGTCGTCACGGCTATACTAACAGTACTTAATGAATAGTTC

     730       740       750       760       770       780       790      800
ATTAGATGGTGCTAGAAACGTTCAAATCCATGGCGTTGGACACATCGGCCTTCTGTACAGCAGCCAAGTCAACAGCCTGA
TAATCTACCACGATCTTTGCAAGTTTAGGTACCGCAACCTGTGTAGCCGGAAGACATGTCGTCGGTTCAGTTGTCGGACT

     810       820       830       840       850       860       870      880
TTAAAGAAGGGCTGAACGGCGGGGGGCCAGAATACGAATTAATGAAAAACAAAACCTTGAAGAATGCTATTCTTCGAGGTT
AATTTCTTCCCGACTTGCCGCCCCCGGTCTTATGCTTAATTACTTTTTGTTTTGGAACTTCTTACGATAAGAAGCTCCAA

     890       900       910       920       930       940       950      960
ATTCCGCTTTCGGCACAATGGTTTTCGCAGCCGTATCGTGAACGGTTTGTTTTTTCTTCGTAAATGCGGCAGTCAAATAG
TAAGGCGAAAGCCGTGTTACCAAAAGCGTCGGCATAGCACTTGCCAAACAAAAAAGAAGCATTTACGCCGTCAGTTTATC

     970       980       990      1000      1010      1020      1030     1040
ATCAGGCGGGAGAACACATGCACCCACGCTATCAGGTAACGGACAATGGCTTGCGGGAAGGATATTTTTTTATATGTTTC
TAGTCCGCCCTCTTGTGTACGTGGGTGCGATAGTCCATTGCCTGTTACCGAACGCCCTTCCTATAAAAAAAATATACAAAG

    1050      1060      1070      1080      1090      1100      1110     1120
GTCCCTCACGATTTGCAGCCCGATGATTTTTTTTGCCCAGTGTGCCCTTCCAATTTGTCAGCGGCATCAGCAAAGGGTACA
CAGGGAGTGCTAAACGTCGGGCTACTAAAAAAAACGGGTCACACGGGAAGGTTAAACAGTCGCCGTAGTCGTTTCCCATGT
```

```
            1130      1140      1150      1160      1170      1180      1190      1200
CAATCAGCATCAATATGGCGACAATAATGACACCGGCGGACCCATCGCCAAACGTAAATCCGGCTGCCAAAATCACTGCT
GTTAGTCGTAGTTATACCGCTGTTATTACTGTGGCCGCCTGGGTAGCGGTTTGCATTTAGGCCGACGGTTTTAGTGACGA

            1210      1220      1230      1240      1250      1260      1270      1280
GCGGCAATGATTACATCAAGTAAAAGAGCGCAGGCGCGCAGCATGAAACCAGCTAGTTCCAATAGAAACACTCCTTAAAA
CGCCGTTACTAATGTAGTTCATTTTCTCGCGTCCGCGCGTCGTACTTTGGTCGATCAAGGTTATCTTTGTGAGGAATTTT

            1290      1300      1310      1320      1330      1340      1350      1360
TGTTAAATAAACACCTAATGATTGTAAAAAAGAAGGGCCAAAGTGGGAATAGGTGATAAGCCTTAAATCACAAAAGTTGG
ACAATTTATTTGTGGATTACTAACATTTTTTCTTCCCGGTTTCACCCTTATCCACTATTCGGAATTTAGTGTTTTCAACC

            1370      1380      1390      1400      1410      1420      1430      1440
CGAAAATGCCATAGGTAAA TGGCATAATCAGCCAGCTTATCACATTACCAAATTCTTTTTTAGCCCGAAACCAAGCCCT
GCTTTTACGGTATCCATTTAACCGTATTAGTCGGTCGAATAGTGTAATGGTTTAAGAAAAAATCGGGCTTTGGTTCGGGA

            1450      1460      1470         EcoRI    1490      1500      1510      1520
CAGAAGTTATTTTTGTTAAAATAGAAAAGTTACAACAGAATTCGGAGGGGTTTATTGTGGGAAAAGCGAAACGAAATGCCC
GTCTTCAATAAAAACAATTTTATCTTTTCAATGTTGTCTTAAGCCTCCCAAATAACACCCTTTTCGCTTTGCTTTACGGG

            1530      1540      1550      1560      1570      1580      1590      1600
CTTGCCCATGCGGCAGCGGCAAGAAATATAAAAAAATGCTGCGGAAGTAAAGTTGTCGACTTCCCGGCGGAACTAGCGGCA
GAACGGGTACGCCGTCGCCGTTCTTTATATTTTTTACGACGCCTTCATTTCAACAGCTGAAGGGCCGCCTTGATCGCCGT

            1610      1620      1630      1640      1650      1660      1670      1680
AAAGAAGCGAAACAAATTCAGGAAGACTTAGTGGAGTATGCCTTCACAGTACATAGAGAAAGCATTTCAGGCTTTATCAA
TTTCTTCGCTTTGTTTAAGTCCTTCTGAATCACCTCATACGGAAGTGTCATGTATCTCTTTCGTAAAGTCCGAAATAGTT

            1690      1700      1710      1720      1730      1740      1750      1760
CCAGCATGATTTTCTTTCTGCTATGGACAGACAGACGAAAGACATCAGCGTATTTAACTTAGGAATCTGGGGAATCCTCC
GGTCGTACTAAAAGAAAGACGATACCTGTCTGTCTGCTTTCTGTAGTCGCATAAATTGAATCCTTAGACCCCTTAGGAGG

            1770      1780      1790      1800      1810      1820      1830      1840
TCCACCCGCTTGCTGGTGAGAAGACAGTCTTCGAAGAGTACCTTCGGAAAAAAGGCGATTCGATCACTCGA
AGGTGGGCGAACGACCACTCTTCTGTCAGAAGCTTCTCATGGAAGCCTTTTTTTCCGCTAAGCTAGTGAGCT
```

FIGURE III